# EUROPEAN PATENT SPECIFICATION

(11) **EP 4 295 308 B1**
(45) Date of publication and mention of the grant of the patent: **18.11.2026**
(21) Application number: 22756813.6
(22) Date of filing: 16.02.2022
(51) Int. Cl.: G06T 7/11, G06T 7/90, G06T 7/187, G06T 7/194, G06V 10/10, G06T 1/00, G06V 10/20, G06N 3/02, G16H 70/40

(54) **METHODS, SYSTEMS, AND COMPUTER PROGRAM PRODUCT FOR VALIDATING DRUG PRODUCT PACKAGE CONTENTS BASED ON CHARACTERISTICS OF THE DRUG PRODUCT PACKAGING SYSTEM**
VERFAHREN, SYSTEME UND COMPUTERPROGRAMMPRODUKT ZUR VALIDIERUNG DES INHALTS EINER ARZNEIMITTELPRODUKTVERPACKUNG AUF BASIS DER EIGENSCHAFTEN DES ARZNEIMITTELPRODUKTVERPACKUNGSSYSTEMS
PROCÉDÉS, SYSTÈMES ET PRODUIT-PROGRAMME INFORMATIQUE POUR VALIDER DES CONTENUS D'UN EMBALLAGE DE MÉDICAMENT SUR LA BASE DE CARACTÉRISTIQUES DU SYSTÈME D'EMBALLAGE DE MÉDICAMENT

(30) Priority: 18.02.2021 US 202163150820 P
(43) Date of publication of application: 27.12.2023
(73) Proprietor: Parata Systems, LLC, Durham, NC 27703 (US)
(72) Inventor: BUGAY, John Alfred, Eden, Utah 84310 (US); JENKINS, Todd Martin, Raleigh, North Carolina 27613 (US); LEWIS, Russell F., Dallas, Texas 75204 (US); MARTIN, Corey Spencer, Raleigh, North Carolina 27606 (US); RAY, Abhishek, Kolkata 700156 (IN); SWANSON, Arthur F., Cary, North Carolina 27518 (US); XU, Rongkai, Morganville, New Jersey 07751 (US)
(74) Representative: Müller-Boré & Partner Patentanwälte PartG mbB
(86) International application number: PCT/US2022/016554
(87) International publication number: WO 2022/177954

(56) References cited:
- KR-A- 20170 054 529
- KR-B1- 101 744 123
- KR-B1- 101 943 217
- KR-B1- 102 009 721
- US-A1- 2014 033 644
- US-A1- 2015 302 255
- CHUNG SHENG-LUEN ET AL: "End-to-end identification of pharmaceutical blister packages based on one-side handheld images", 2020 INTERNATIONAL CONFERENCE ON SYSTEM SCIENCE AND ENGINEERING (ICSSE), IEEE, 31 August 2020 (2020-08-31), pages 1 - 5, XP033840528, DOI: 10.1109/ICSSE50014.2020.9219265
- ZDENEK JAN ET AL: "Erasing Scene Text with Weak Supervision", 2020 IEEE WINTER CONFERENCE ON APPLICATIONS OF COMPUTER VISION (WACV), IEEE, 1 March 2020 (2020-03-01), pages 2227 - 2235, XP033771188, DOI: 10.1109/WACV45572.2020.9093544

## Description

### BACKGROUND

The present disclosure relates generally to the packaging of drug products, and, in particular, to methods, systems, and computer program products for validating drug product package contents based on characteristics of the drug product packaging system.

Drug product packaging systems may be used in facilities, such as pharmacies, hospitals, long term care facilities, and the like to dispense medications to fill prescriptions. These drug product packaging systems may include systems designed to package medications in various container types including, but not limited to, pouches, vials, bottles, blistercard, and strip packaging. Strip packaging is a type of packaging wherein medications are packaged in individual pouches for administration on a specific date and, in some cases, at a specific time. Typically, individual pouches are removably joined together and often provided in rolls. The pouches can be separated from the roll when needed.

Some types of drug product packages, such as pouches and blistercards, for example, may contain content printed thereon, such as Personal Health Information (PHI), manufacturer information, e.g., logos, names, contact information, etc., and/or other details about the content of the drug product package, such as number of drug products, drug product names, dosing times, dosing strengths, barcodes, and the like. Such content on the surface of the drug product package may make it more difficult to validate the content of the drug product package through imaging of the drug product package.

Moreover, different drug product packaging systems may have different characteristics with respect to the types of materials used in packaging the drug products and/or the validation system used in validating the contents of a drug product package. These variations between different drug product packaging systems may further complicate the evaluation of images taken of packaged drug products to confirm the contents thereof.

"End-to-end identification of pharmaceutical blister packages based on one-side handheld images," by Chung et al., describes image-based identification of handheld pharmaceutical blister packages relying on only one side images and requiring only one end-to-end deep learning network.

U.S. Patent Publication No. 2014/033644 to Amano et al., describes a medicine inspection device that includes an inspection unit for inspecting a medicine disposed on the inspection unit. The medicine inspection device is characterized by operating a vibrator so as to provide vibration in inspection areas chosen based on a detection result.

"Erasing Scene Text with Weak Supervision", Jan Zdenek et al., describes a method for scene text erasing using a separately trained scene text detector and an inpainting network.

### SUMMARY

The claimed invention is defined by the independent claims. Embodiments are set out in the dependent claims.

A method comprises, receiving an image of a drug product package that contains one or more drug products therein; detecting, using an artificial intelligence engine, characteristics of the image that are associated with a drug product packaging system; and generating a modified image of the drug product package based on characteristics of the drug product packaging system.

In embodiments, the characteristics of the drug product packaging system comprise one or more image capture light source characteristics, one or more image capture surface characteristics, one or more packaging material characteristics, and/or one or more camera characteristics.

In other embodiments, the one or more light source characteristics comprise strength of an image capture light source, intensity of the image capture light source, and/or location of the image capture light source.

In still other embodiments, the one or more image capture surface characteristics comprise background location and/or background color.

In still other embodiments, the one or more packaging material characteristics comprise packaging material transparency, packaging material shadow, labeling color, packaging material color, and/or packaging material hot spot.

In still other embodiments, the one or more camera characteristics comprise camera number, camera position, camera resolution, and/or camera image type.

In still other embodiments, the image is a first image, the drug product package is a first drug product package, and the modified image is a first modified image. Detecting, using the artificial intelligence engine, the characteristics of the first image comprises detecting, using the artificial intelligence engine, characteristics of the first image that are associated with a first one of a plurality of drug product packaging systems. Generating the first modified image comprises generating the first modified image of the first drug product package based on characteristics of the first one of the plurality of drug product packaging systems.

In still other embodiments, the method further comprises receiving a second image of a second drug product package that contains one or more drug products therein; detecting, using the artificial intelligence engine, characteristics of the second image that are associated with a second one of the plurality of drug product packaging systems; and generating a second modified image of the second drug product package based on characteristics of the second one of the plurality of drug product packaging systems.

In still other embodiments, the method further comprises detecting, using an artificial intelligence engine, labeling content on a surface of the drug product package. Generating the modified image of the drug product package comprises generating a modified image of the drug product package that has the labeling content removed from the surface thereof.

In still other embodiments, the artificial intelligence engine is a first artificial intelligence engine and the modified image is a first modified image. The method further comprises receiving order information for the one or more drug products and an identifier for the drug product package; detecting, using a second artificial intelligence engine, individual ones of the one or more drug products in the first modified image; and generating a second modified image of the drug product package that includes indicia that distinguish between the individual ones of the one or more drug products and associate the one or more drug products with the order information and the identifier for the drug product package.

In still other embodiments, the order information comprises names for the one or more drug products in the drug product package. The method further comprises identifying, using a third artificial intelligence engine, at least some of the one or more drug products in the second modified image based on the names for the one or more drug products. The names are associated with drug product attributes in a reference database.

In still other embodiments, the method further comprises performing gamma correction on the image of the drug product package responsive to receiving the image of the drug product package to generate a gamma corrected image of the drug product package; performing gaussian blur denoising on the gamma corrected image of the drug product package to generate a reduced noise image of the drug product package; and performing automatic image thresholding on the reduced noise image of the drug product package to generate a foreground-background separated image of the drug product package. Detecting, using the artificial intelligence engine, the labeling content comprises detecting, using the artificial intelligence engine, the labeling content on the surface of the foreground-background separated image of the drug product package.

A system comprises a processor and a memory coupled to the processor and comprising computer readable program code embodied in the memory that is executable by the processor to perform operations comprising: receiving an image of a drug product package that contains one or more drug products therein; detecting, using an artificial intelligence engine, characteristics of the image that are associated with a drug product packaging system; and generating a modified image of the drug product package based on characteristics of the drug product packaging system.

In further embodiments, the characteristics of the drug product packaging system comprise one or more image capture light source characteristics, one or more image capture surface characteristics, one or more packaging material characteristics, and/or one or more camera characteristics.

In still further embodiments, the one or more light source characteristics comprise strength of an image capture light source, intensity of the image capture light source, and/or location of the image capture light source.

In still further embodiments, the one or more image capture surface characteristics comprise background location and/or background color.

In still further embodiments, the one or more packaging material characteristics comprise packaging material transparency, packaging material shadow, labeling color, packaging material color, and/or packaging material hot spot.

In still further embodiments, the one or more camera characteristics comprise camera number, camera position, camera resolution, and/or camera image type.

In still further embodiments, the image is a first image, the drug product package is a first drug product package, and the modified image is a first modified image. Detecting, using the artificial intelligence engine, the characteristics of the first image comprises detecting, using the artificial intelligence engine, characteristics of the first image that are associated with a first one of a plurality of drug product packaging systems. Generating the first modified image comprises generating the first modified image of the first drug product package based on characteristics of the first one of the plurality of drug product packaging systems.

In still further embodiments, the operations further comprise receiving a second image of a second drug product package that contains one or more drug products therein; detecting, using the artificial intelligence engine, characteristics of the second image that are associated with a second one of the plurality of drug product packaging systems; and generating a second modified image of the second drug product package based on characteristics of the second one of the plurality of drug product packaging systems.

A computer program product comprises a non-transitory computer readable storage medium comprising computer readable program code embodied in the medium that is executable by a processor to perform operations comprising: receiving an image of a drug product package that contains one or more drug products therein; detecting, using an artificial intelligence engine, characteristics of the image that are associated with a drug product packaging system; and generating a modified image of the drug product package based on characteristics of the drug product packaging system.

Other methods, systems, articles of manufacture, and/or computer program products according to embodiments of the inventive concept will be or become apparent to one with skill in the art upon review of the following drawings and detailed description. It is intended that all such additional systems, methods, articles of manufacture, and/or computer program products be included within this description, be within the scope of the present inventive subject matter and be protected by the accompanying claims.

### BRIEF DESCRIPTION OF THE DRAWINGS

Other features of embodiments will be more readily understood from the following detailed description of specific embodiments thereof when read in conjunction with the accompanying drawings, in which:
FIG. 1 is a block diagram that illustrates a communication network including an Artificial Intelligence (AI) assisted drug product package analysis system that can account for variations between different drug product packaging systems in accordance with some embodiments of the inventive concept;
FIG. 2 is a block diagram of the AI assisted drug product package analysis system of FIG. 1 in accordance with some embodiments of the inventive concept;
FIG. 3 is a block diagram of a convolutional neural network for detecting labeling content of the surface of a drug product package and can account for variations between different drug product packaging systems in accordance with some embodiments of the inventive concept;
FIG. 4 is a block diagram that illustrates drug product package image pre-processing in accordance with some embodiments of the inventive concept;
FIG. 5 is a block diagram of a skip connection arrangement between convolutional layers of the convolutional neural network of FIG. 3 in accordance with some embodiments of the inventive concept;
FIGS. 6 - 11 are flowcharts that illustrate operations for performing drug product package analysis while accounting for variations between different drug product packaging systems in accordance with some embodiments of the inventive concept;
FIG. 12 is a data processing system that may be used to implement one or more servers in the AI assisted drug product package analysis system of FIG. 1 in accordance with some embodiments of the inventive concept; and
FIG. 13 is a block diagram that illustrates a software/hardware architecture for use in the AI assisted drug product package analysis system of FIG. 1 in accordance with some embodiments of the inventive concept.

### DETAILED DESCRIPTION

In the following detailed description, numerous specific details are set forth to provide a thorough understanding of embodiments of the present inventive concept. However, it will be understood by those skilled in the art that the present invention may be practiced without these specific details. In some instances, well-known methods, procedures, components, and circuits have not been described in detail so as not to obscure the present inventive concept. It is intended that all embodiments disclosed herein can be implemented separately or combined in any way and/or combination. Aspects described with respect to one embodiment may be incorporated in different embodiments although not specifically described relative thereto. That is, all embodiments and/or features of any embodiments can be combined in any way and/or combination.

As used herein, the term "data processing facility" includes, but it is not limited to, a hardware element, firmware component, and/or software component. A data processing system may be configured with one or more data processing facilities.

The term "drug product packaging system," as used herein, refers to any type of pharmaceutical dispensing system including, but not limited to, automated systems that fill vials, bottles, containers, pouches, blistercards, or the like with drug product, semi-automated systems that fill vials, bottles, containers, pouches, blistercards, or the like with drug product, and any combination of automated and semi-automated systems for filling a drug product package with drug product. Drug product packaging system also includes packaging systems for pharmaceutical alternatives, such as nutraceuticals and/or bioceuticals.

The terms "pharmaceutical" and "medication," as used herein, are interchangeable and refer to medicaments prescribed to patients either human or animal. A pharmaceutical or medication may be embodied in a variety of ways including, but not limited to, pill form capsule form, tablet form, and the like.

The term "drug product" refers to any type of medicament that can be packaged within a vial, bottle, container, pouch, blistercard, or the like by automated and semi-automated drug product packaging systems including, but not limited to, pills, capsules, tablets, caplets, gel caps, lozenges, and the like. Drug product also refers to pharmaceutical alternatives, such as nutraceuticals and/or bioceuticals. Example drug product packaging systems including management techniques for fulfilling packaging orders are described in U. S. Patent No. 10,492,987.

The term "drug product package" refers to any type of object that can hold a drug product including, but not limited to, a vial, bottle, container, pouch, blistercard, or the like.

The term "National Drug Code (NDC)" may be used to represent both NDC information and "Drug Identification Number (DIN)" information.

Embodiments of the inventive concept are described herein in the context of a drug product packaging analysis engine that includes one or more machine learning engines and artificial intelligence (AI) engines. It will be understood that embodiments of the inventive concept are not limited to particular implementations of the drug product analysis engine and various types of AI systems may be used including, but not limited to, a multi-layer neural network, a deep learning system, a natural language processing system, and/or computer vision system Moreover, it will be understood that the multi-layer neural network is a multi-layer artificial neural network comprising artificial neurons or nodes and does not include a biological neural network comprising real biological neurons. Embodiments of the inventive concept may be implemented using multiple AI systems or may be implemented by combining various functionalities into fewer or a single AI system.

Some embodiments of the inventive concept stem from a realization that when validating the contents of a drug product package, such as a pouch or blistercard, for example, different drug product packaging systems may have different characteristics that can affect the validation process due to, for example, differences in the physical packaging being used as well as the image capture system used by the different drug product packaging systems to capture images of the drug product packages. For example, the characteristics of the drug product packaging system may comprise one or more image capture light source characteristics, one or more image capture surface characteristics, one or more packaging material characteristics, and/or one or more camera characteristics. The one or more light source characteristics may comprise, for example, strength of an image capture light source, intensity of the image capture light source, and/or location of the image capture light source. Ambient light may also affect the light source characteristics based on the construction of the image capture system associated with the drug product packaging system. The one or more image capture surface characteristics may comprise background location and/or background color. The one or more packaging material characteristics may comprise packaging material transparency, packaging material shadow, labeling color, packaging material color, and/or packaging material hot spot. Colors may be used on a drug product package to differentiate drug product and each color may represent a different drug class. For example, blue labels may be used to identify opioids, fluorescent red may be used to identify neuromuscular blockers, yellow may be used to identify induction agents, orange may be used to identify tranquilizers, violet may be used to identify vasopressors, and green may be used to identify anticholinergics. Blue vials may be used to identify drug products that need to be kept out of reach of children. Blue pill bottles may be made from polyethylene material that is generally long lasting. The pill bottles may secure different quantities and sizes of drug products for safe transport and storage. Pill bottles may be translucent orange to mimic amber colored bottles that were used historically. The orange coloring may reduce damage to the drug products contained therein due to UV light. The one or more camera characteristics comprise camera number, camera position, camera resolution, and/or camera image type. Profile information including the above-described characteristics may be stored for each of the drug product packaging systems to aid in drug product packaging system specific image recognition. The profile information for each drug product packaging system may not be static and may evolve over time due to changes in the external environment, parts changes/wear, and the like. Thus, as the drug product packaging system's characteristics evolve, the AI system may likewise be trained to recognize the evolving characteristics over time. Conventional drug product package content validation systems are typically tailored to a particular drug product packaging system. By taking into account variations in the packaging and/or the image capture system used in different drug product packaging systems in validating the contents of the drug product packages through use of an AI system, the accuracy of the drug product packaging validation can be improved. Moreover, the drug product package content validation may be moved in whole or in part into a network location, such as the cloud, allowing a pharmacy or other drug product packaging facility to access the AI based drug product package validation system that has been trained on multiple types of drug product packaging systems. As new drug product packaging systems are developed, the AI system may be trained to recognize the particular characteristics of the new drug product packaging system. In other embodiments, some facilities may desire to run the drug product package content validation locally rather than over a network, such as via the cloud. In other embodiments, the AI system may be modified to run locally at a particular pharmacy or drug product packaging facility and may be pared down to support the particular drug product packaging system used at that pharmacy or facility without supporting other drug product packaging systems.

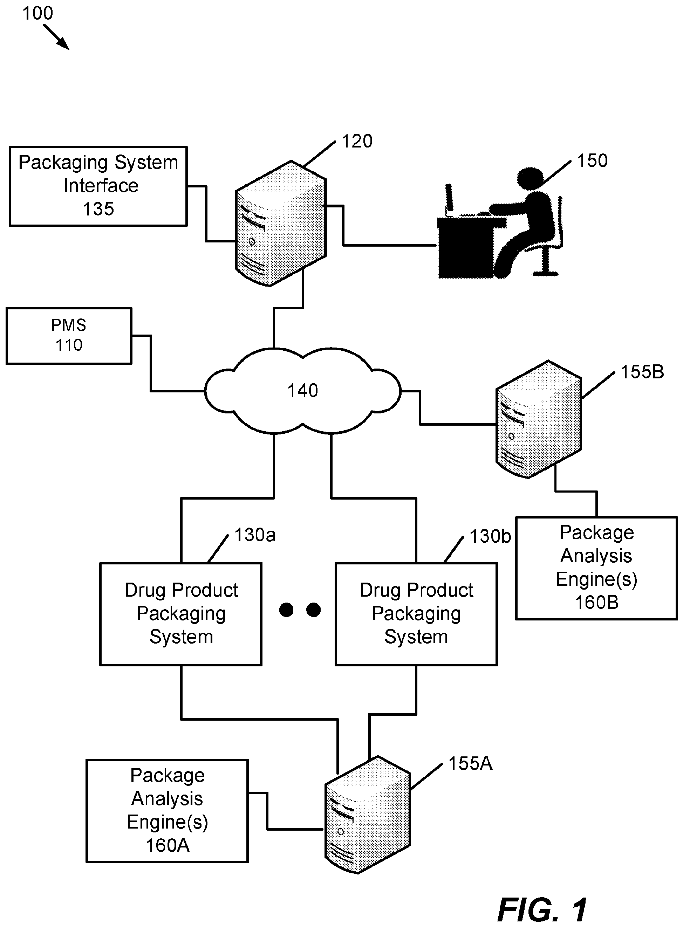

Referring to FIG. 1, a communication network 100a including an AI assisted drug product package analysis system that can account for variations between different drug product packaging systems, in accordance with some embodiments of the inventive concept, comprises a pharmacy management system (PMS) or host system 110, a packaging system server 120, a package analysis engine(s) server 155, and one or more drug product packaging systems 130a and 130b that are coupled via a network 140 as shown.
The PMS system 110 may be configured to manage and fill prescriptions for customers. As used herein, PMS systems may be used in pharmacies or may be used generally as batch-generating systems for other applications, such as dispensing nutraceuticals or bioceuticals. The PMS system 110 may be associated with a variety of types of facilities, such as pharmacies, hospitals, long term care facilities, and the like. The PMS system or host system 110 may be any system capable of sending a valid prescription to the one or more product packaging systems 130a and 130b. The packaging system server 120 may include a packaging system interface module 135 and may be configured to manage the operation of the drug product packaging systems 130a and 130b. For example, the packaging system server 120 may be configured to receive packaging orders from the PMS system 110 and to identify which of the drug product packaging systems 130a and 130b should be used to package particular individual orders or batches of orders. In some embodiments, an AI system may be used to facilitate intelligent routing of drug product packaging orders to specific drug product packaging systems as described, for example, in U. S. Patent Application No. 17/510,635, filed October 26, 2021 entitled ORGANIZATION OF SCRIPT PACKAGING SEQUENCE AND PACKAGING SYSTEM SELECTION FOR DRUG PRODUCTS USING AN ARTIFICIAL INTELLIGENCE ENGINE.

In addition, the packaging system server 120 may be configured to manage the operations of the drug product packaging systems 130a and 130b. For example, the packaging system server 120 may be configured to manage the inventory of drug product available through each of the drug product packaging systems 130a and 130b, to manage the drug product dispensing canisters assigned or registered to one or more of the drug product packaging systems 130a and 130b, to manage the operational status generally of the drug product packaging systems 130a and 130b, and/or to manage reports regarding the status (e.g., assignment, completion, etc.) of packaging orders, drug product inventory, order billing, and the like. A user 150, such as a pharmacist or pharmacy technician, may communicate with the packaging system server 120 using any suitable computing device via a wired and/or wireless connection. Although the user 150 is shown communicating with the packaging system server 120 via a direct connection in FIG. 1, it will be understood that the user 150 may communicate with the packaging system server 120 via one or more network connections. The user 150 may interact with the packaging system server 120 to approve or override various recommendations made by the packaging system server 120 in operating the drug product packaging systems 130a and 130b. The user 150 may also initiate the running of various reports as described above for the drug product packaging systems 130a and 130b. Although only two drug product packaging systems 130a and 130b are shown in FIG. 1, it will be understood that more than two drug product packaging systems may be managed by the packaging system server 120. The drug product packaging systems 130a and 130b may be the same type of drug product packaging system or may be different types of drug product packaging systems. Moreover, the drug product packaging systems 130a and 130b may be in the same facility or in different facilities. As a result, the drug product packaging systems 130a and 130b may have different characteristics that can affect the validation process of the content of drug product packages due to, for example, differences in the physical packaging being used as well as the image capture system used by the different drug product packaging system to capture images of the drug product packages.

The AI assisted drug product package analysis system may include the package analysis engine(s) servers 155A and/or 155B, which includes package analysis engine(s) modules 160A and 160B to facilitate validation of the contents of a drug product package while taking into account variations between characteristics of the different drug product packaging systems 130a and 130b. In accordance with various embodiments of the inventive concept, the drug product package analysis service may be provided over a network connection, such as a cloud service, by way of the package engine analysis engine server 155B and the package analysis engine module 160B and/or may be provided locally, for example, at a pharmacy or drug product packaging facility by way of the package engine analysis engine server 155A and the package analysis engine module 160A. As will be described herein, the AI assisted drug product package analysis service may be provided as a composite service in which an AI system is trained to account for multiple types of drug product packaging systems or may be provided as a targeted service to account for a single type of drug product packaging system. A pharmacy or other drug product packaging facility may wish to run the AI assisted drug product package analysis service locally, which is targeted to a specific drug product packaging system used in the pharmacy or facility. In other embodiments, a pharmacy or drug product packaging facility may wish to access the drug product package analysis service as a cloud service, in which the AI system is trained to account for the varying characteristics of multiple types of drug product packaging systems as the pharmacy or drug product packaging facility may use multiple types of drug product packaging systems or may not wish to setup the resources locally to run the drug product package analysis service. The package engine analysis engine server 155B and the package analysis engine module 160B along with the package engine analysis engine server 155A and the package analysis engine module 160A will be collectively referred to herein as the package analysis engine server 155 and the package analysis engine module 160. The package analysis engine server 155 and the package analysis engine module 160 may facilitate AI assisted drug product package analysis to validate the drug product package contents while accounting for variations in characteristics of drug product packaging systems for drug product packaging systems in a same physical locations and across multiple physical locations.

The package analysis engine(s) server 155 and package analysis engine(s) module 160 may represent one or more AI systems that may be configured to generate a modified image of a drug product package based on the characteristics of the drug product packaging system, to generate modified images of drug product packages with labeling content removed from one or more surfaces thereof, to detect in a drug product package image individual ones of one or more drug products contained in the drug product package, and/or to identify these drug products that have been detected in the drug product package image. In accordance with some embodiments of the inventive concept, the characteristics of the drug product packaging system may comprise one or more image capture light source characteristics, one or more image capture surface characteristics, one or more packaging material characteristics, and/or one or more camera characteristics. In accordance with various embodiments of the inventive concept, the labeling content can be removed from any surface on the drug product package including multiple surfaces of the drug product package, such as the top, bottom, and sides of vials, front and back surfaces of pouches and blister packs, and the like.

It will be understood that the division of functionality described herein between the packaging system server 120/packaging system interface module 135 and the package analysis engine(s) server 155/package analysis engine(s) module 160 is an example. Various functionality and capabilities can be moved between the packaging system server 120/packaging system interface module 135 and the package analysis engine(s) server 155/package analysis engine(s) module 160 in accordance with different embodiments of the inventive concept. Moreover, in some embodiments, the packaging system server 120/packaging system interface module 135 and the package analysis engine(s) server 155/package analysis engine(s) module 160 may be merged as a single logical and/or physical entity.

A network 140 couples the drug product packaging systems 130a and 130b, the PMS system 110, the package engine analysis engine server 155B, and the packaging system server 120 to one another. The network 140 may be a global network, such as the Internet or other publicly accessible network. Various elements of the network 140 may be interconnected by a wide area network, a local area network, an Intranet, and/or other private network, which may not be accessible by the general public. Thus, the communication network 140 may represent a combination of public and private networks or a virtual private network (VPN). The network 140 may be a wireless network, a wireline network, or may be a combination of both wireless and wireline networks. In some embodiments, the package analysis engine(s) server 155 may also be coupled to the network 140.

The AI assisted drug product package analysis service provided through the package analysis engine(s) server 155, and package analysis engine(s) module 160, in some embodiments, may be implemented as a cloud service, such as through the package engine analysis engine server 155B and the package analysis engine module 160B. In some embodiments, the AI assisted drug product package analysis service may be implemented as a Representational State Transfer Web Service (RESTful Web service).

Although FIG. 1 illustrates an example communication network that includes AI assisted drug product package analysis systems that can account for variations between different drug product packaging systems, it will be understood that embodiments of the inventive subject matter are not limited to such configurations, but are intended to encompass any configuration capable of carrying out the operations described herein.

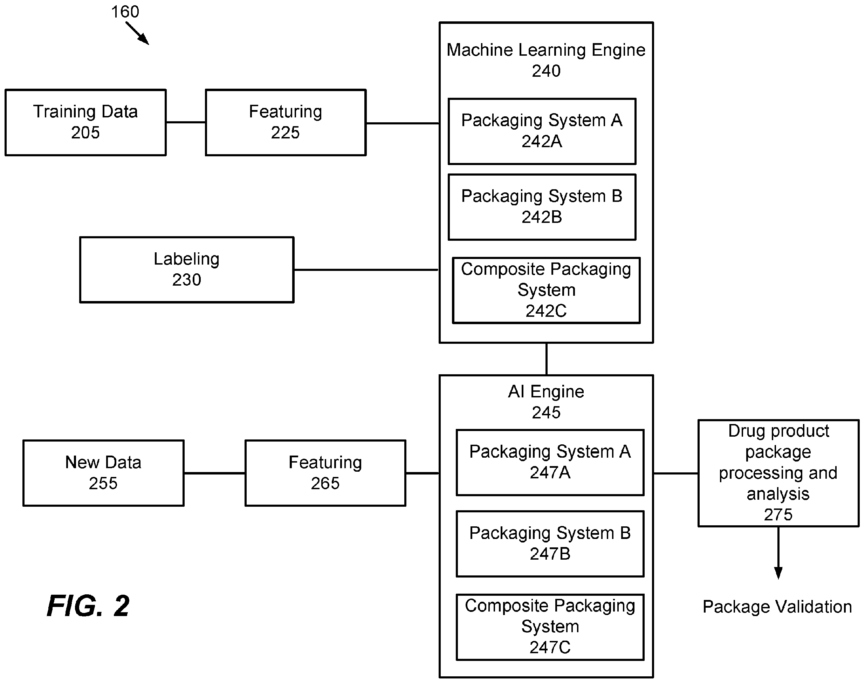

As described above, the package analysis engine(s) server 155 and package analysis engine(s) module 160 may represent one or more AI systems that may be configured to account for variations between different drug product packaging systems, to generate modified images of drug product packages with labeling content removed from the surfaces thereof, to detect in a drug product package image individual ones of one or more drug products contained in the drug product package, and/or to identify these drug products that have been detected in the drug product package image. FIG. 2 is a block diagram of the package analysis engine(s) module 160 for implementing an AI system, such as a machine learning system, that can be used to detect in a drug product package image individual ones of one or more drug products contained in the drug product package, and/or to identify these drug products that have been detected in the drug product package image while accounting for variations in characteristics of the drug product packaging systems used to package the drug products. The AI system of FIG. 2 may be implemented as a single AI system to detect in a drug product package image individual ones of one or more drug products contained in the drug product package and to identify these drug products that have been detected in the drug product package image while accounting for variations in characteristics of the drug product packaging systems used to package the drug products. In other embodiments, the architecture of the AI system of FIG. 2 may be duplicated to form separate AI systems to detect in a drug product package image individual ones of one or more drug products contained in the drug product package and to identify these drug products that have been detected in the drug product package image while accounting for variations in characteristics of the drug product packaging systems used to package the drug products, respectively. As shown in FIG. 2, the package analysis engine(s) module 160 may include both training modules and modules used for processing new data on which to detect and/or identify drug products in a drug product package image while accounting for variations in characteristics of the drug product packaging systems used to package the drug products. The modules used in the training portion of the package analysis engine(s) module 160 include the training data module 205, the featuring module 225, the labeling module 230, and the machine learning engine 240.

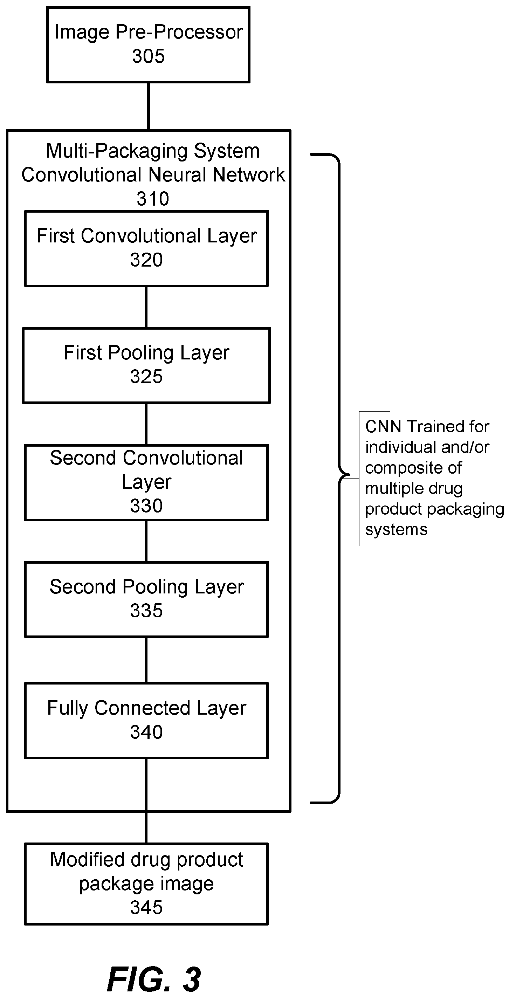

The training data 205 may comprise one or more images of a drug product package that each contain one or more drug products therein. The drug product package(s) may include labeling content on a surface thereof, which may include, but is not limited to, commercial marketing information, patient identification information and/or personal healthcare information (PHI). The commercial marketing information may include, for example, a logo and/or a business name. The patient identification information may include, for example, a patient name, a patient phone number, a patient address, and/or a patient identification number. The personal health care information may include, for example, names of the one or more drug products contained in the drug product package, a time of administration for each of the one or more drug products, one or more barcodes associated with the one or more drug products, a prescription order, a patient account, an identification number, and/or other information. In some embodiments, the drug product package image may be modified, such that at least a portion of the labeling content contained on the surface thereof is removed through use of an AI system, such as a neural network, described below with reference to FIG. 3. In some embodiments, to detect in a modified drug product package image with at least a portion of the labeling content on the surface thereof removed individual ones of one or more drug products contained in the drug product package, the training data 205 may further include order information for the one or more drug products contained in the drug product package and/or an identifier for the drug product package. In further embodiments, to identify these drug products that have been detected in the drug product package image the order information included in the training data 205 may include names for the one or more drug products in the drug product package. Different drug product packaging systems may differ from one another with respect to characteristics that can affect the detection/identification of one or more drug products in a drug product package and/or the removal of labeling content from a drug product package. Thus, the training data 205 may include information related to characteristics of the one or more images that are associated with the drug product packaging system used to package the drug product. These characteristics may comprise one or more image capture light source characteristics, one or more image capture surface characteristics, one or more packaging material characteristics, and/or one or more camera characteristics. The one or more light source characteristics may comprise, for example, strength of an image capture light source, intensity of the image capture light source, and/or location of the image capture light source. The one or more image capture surface characteristics may comprise background location and/or background color. The one or more packaging material characteristics may comprise packaging material transparency, packaging material shadow, labeling color, packaging material color, and/or packaging material hot spot. The one or more camera characteristics comprise camera number, camera position, camera resolution, and/or camera image type. The training data 205 may further include reference drug product package images including information identifying the drug product package characteristics and/or the contents contained therein. These known reference packages may serve as a baseline for the machine learning engine 240 to learn and the AI engine 245 to recognize deviations in the characteristics of different drug product packaging systems relative to known reference images.

The featuring module 225 is configured to identify the individual independent variables that are used by the package analysis engine(s) module 160 to detect and/or identify one or more drug products in, for example, a drug product package image having had the labeling content removed, which may be considered dependent variable(s), while accounting for the characteristics of the drug product packaging system used in packaging the one or more drug products. For example, the training data 205 may be generally unprocessed or formatted and include extra information in addition to drug product and/or drug product packaging information. For example, the training data 205 may include account codes, business address information, and the like, which can be filtered out by the featuring module 225. The features extracted from the training data 205 may be called attributes and the number of features may be called the dimension. The labeling module 230 may be configured to assign defined labels to the training data and to the detected and/or identified drug products to ensure a consistent naming convention for both the input features and the generated outputs. The machine learning engine 240 may process both the featured training data 205, including the labels provided by the labeling module 230, and may be configured to test numerous functions to establish a quantitative relationship between the featured and labeled input data and the generated outputs. The machine learning engine 240 may use modeling techniques to evaluate the effects of various input data features on the generated outputs. These effects may then be used to tune and refine the quantitative relationship between the featured and labeled input data and the generated outputs. The tuned and refined quantitative relationship between the featured and labeled input data generated by the machine learning engine 240 is output for use in the AI engine 245. The machine learning engine 240 may be referred to as a machine learning algorithm. As shown in FIG. 2, the machine learning engine 240 may be trained to support multiple individual packaging systems as represented by the packaging system A module 242A and the packaging system B module 242B. In further embodiments, the machine learning engine 240 may be trained to support the analysis of drug product package images from one or more of a plurality of packaging systems as represented by the composite packaging system module 242C.

The modules used to detect in a drug product package image individual ones of one or more drug products contained in the drug product package and/or to identify these drug products that have been detected in the drug product package image based on the characteristics of the drug product packaging system used to generate the drug product package include the new data module 255, the featuring module 265, the AI engine module 245, and the drug product package processing and analysis module 275. The new data 255 may be the same data/information as the training data 205 in content and form except the new data 255 will be used for an analysis of a new drug product package rather than for training purposes. Likewise, the featuring module 265 performs the same functionality on the new data 255 as the featuring module 225 performs on the training data 205. The AI engine 245 may, in effect, be generated by the machine learning engine 240 in the form of the quantitative relationship determined between the featured and labeled input data and the output drug product package content analysis. The AI engine 245 may, in some embodiments, be referred to as an AI model. Similar to the machine learning engine 240, the AI engine 245 supports multiple individual packaging systems as represented by the packaging system A module 247A and the packaging system B module 247B. The machine learning engine 240 may further support the analysis of drug product package images from one or more of a plurality of packaging systems as represented by the composite packaging system module 247C. The AI engine 245 may be configured to generate modified images of a drug product package that includes indicia that distinguish between individual ones of the one or more drug products contained therein while associating the one or more drug products with order information and/or an identifier for the drug product package based on characteristics of the drug product packaging system used to package the one or more drug products. The indicia may be embodied in a variety of ways including, but not limited to, boundary boxes or polygons, circles, an enclosed shape that includes straight and curved surfaces, an enclosed shape that includes only curved surfaces, and/or lines or symbols that demarcate boundaries between the one or more drug products. In some embodiments, the indicia may take a shape that approximates the shape of the drug product. The AI engine 245 may also be configured to identify one or more of the drug products based on their names. The AI engine 245 may use a variety of modeling techniques to detect in a drug product package image individual ones of one or more drug products contained in the drug product package and to identify these drug products that have been detected in the drug product package image based on the characteristics of the drug product packaging system used to package the one or more drug products in accordance with different embodiments of the inventive concept including, but not limited to, a regression technique, a neural network technique, an Autoregressive Integrated Moving Average (ARIMA) technique, a deep learning technique, a linear discriminant analysis technique, a decision tree technique, a naive Bayes technique, a K-nearest neighbors technique, a learning vector quantization technique, a support vector machine technique, and/or a bagging/random forest technique.

The drug product package processing and analysis module 275 may be configured to output the modified drug product package image with the one or more drug products identified by way of indicial, such as boundary boxes, along with names for the one or more drug products to a drug product package validation system.

As described above, the package analysis engine(s) server 155 and package analysis engine(s) module 160 may represent one or more AI systems that may be configured to generate modified images of drug product packages with labeling content removed from the surfaces thereof based on characteristics of the drug product packaging system used in the packaging. FIG. 3 is a block diagram of the package analysis engine(s) module 160 for implementing an AI system, by way of a neural network, that can be used to generate modified images of drug product packages with labeling content removed from the surfaces thereof while accounting for variations between different drug product packaging systems. In the example embodiment of FIG. 3, the neural network is a convolutional neural network. It will be understood, however, that the AI system for removing labeling content from a drug product package image based on characteristics of the drug product packaging system used in the packaging may also be embodied as a fully connected neural network in accordance with other embodiments of the inventive concept. A convolutional neural network may, however, be useful when processing or classifying images due to the large number of pixels and the resulting large number of weights to manage in the neural network layers. A convolutional neural network may reduce the main image matrix to a matrix having a lower dimension in the first layer through convolution, which reduces the number of weights used and reduces the impact on training time.

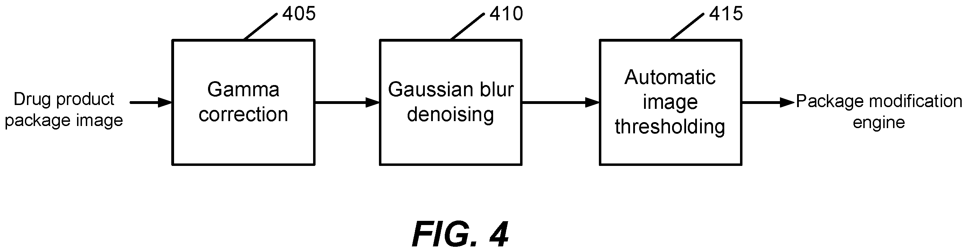

Referring now to FIG. 3, an image pre-processor 305 may receive one or more images of a drug product package that includes labeling content displayed on the surface thereof. As shown in FIG. 4, the image pre-processor may comprise a gamma correction module 405, a gaussian blur denoising module 410, and an automatic image thresholding module 415, which may perform various corrections to the image data including, for example, gamma correction, noise reduction, and/or image segmentation or image thresholding, respectively. Image pre-processing operations, in accordance with some embodiments of the inventive concept are described below. The pre-processed drug product package image, which may be an image represented by a matrix of dimension AxBx3, where the number 3 represents the colors red, green, and blue, may then be provided to the multi-packaging system convolutional neural network 310. According to embodiments described herein, the multi-packaging system convolutional neural network may be a convolutional neural network that is trained to account for characteristics of one or more individual drug product packaging systems when processing drug product package images. The multi-packaging system convolutional neural network 310 may be trained to account for a composite of multiple drug product packaging systems with each packaging system including its own characteristics when processing drug product package images. As shown in FIG. 3, the multi-packaging system convolutional neural network 310 includes first and second convolutional layers 320 and 330 along with first and second pooling layers 325 and 335. Each of the convolutional layers 320 and 330 is a matrix of a dimension smaller than the input matrix and may be configured to perform a convolution operation with a portion of the input matrix having the same dimension. The sum of the products of the corresponding elements is the output of the convolutional layer. The output of each of the convolutional layers may also be processed through a rectified linear unit operation in which any number below 0 is converted to 0 and any positive number is left unchanged. The multi-packaging system convolutional neural network 310 further includes first and second pooling layers 325 and 335. The pooling layers 325 and 335 may each be configured to filter the output of the convolutional layers 320 and 330, respectively, by performing a down sampling operation. The size of the pooling operation or filter is smaller than the size of the input feature map. In some embodiments, it is 2×2 pixels applied with a stride of 2 pixels. This means that the pooling layer will always reduce the size of each feature map by a factor of 2, e.g., each dimension is halved, reducing the number of pixels or values in each feature map to one quarter the size. For example, a pooling layer applied to a feature map of 6×6 (36 pixels) will result in an output pooled feature map of 3×3 (9 pixels). The final output layer is a normal fully-connected neural network layer 340, which gives the output as modified drug product package image 345 with at least a portion of the labeling content on a surface thereof removed.

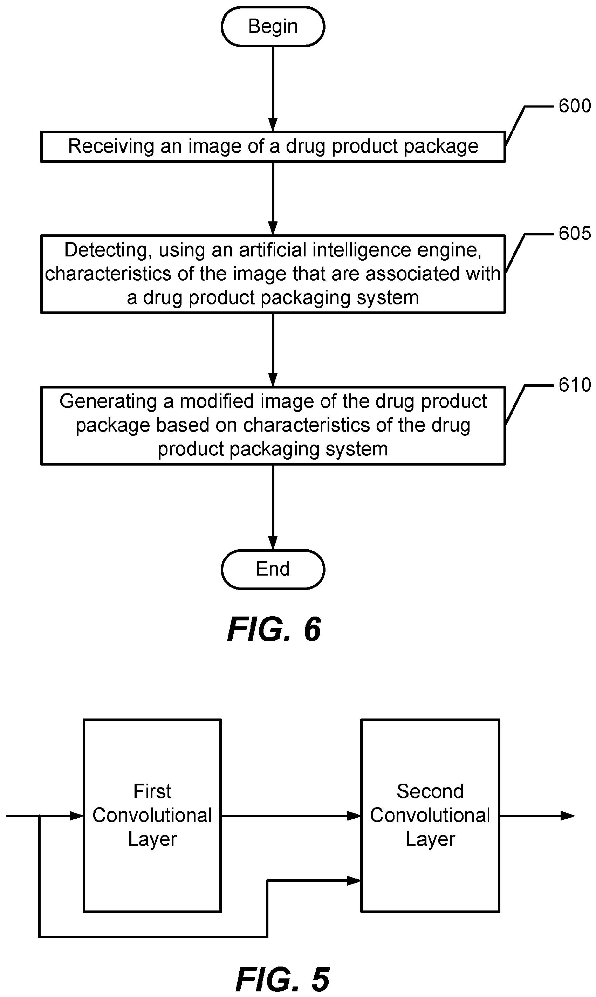

In some embodiments of the inventive concept, the multi-packaging system convolutional neural network 310 may be a residual neural network in which skip connections are used between the convolutional layers 320 and 330. An example of the skip connection is shown in FIG. 5. Specifically, in a skip connection a convolutional neural network involves a convolutional layer receiving as an input both the output of a previous convolutional layer and the input to the previous convolutional layer.

It will be understood that while two convolutional layers 320 and 330 are shown in in the example multi-packaging system convolutional neural network 310 of FIG. 3 for purposes of illustration, a convolutional neural network according to various embodiments of the inventive concept may contain numerous convolutional layers and may exceed 100 layers in some embodiments.

FIGS. 6 - 11 are flowcharts that illustrate operations for performing drug product package analysis including removal of labeling content therefrom to facilitate validation of the contents therein while accounting for variations between different drug product packaging systems in accordance with some embodiments of the inventive concept. Referring to FIG. 6, operations begin at block 600 where the AI engine 245 and/or the multi-packaging system convolutional neural network 310 receives an image of a drug product package that contains one or more drug products therein. In some embodiments, a drug product package may be an outer package that includes one or more drug product packages therein. For example, a large pouch or bag may contain multiple bar coded blister packs, pouches, and/or strips. At block 605, the characteristics of the image that are associated with a drug product packaging system may be detected using the AI engine 245 and/or the multi-packaging system convolutional neural network 310. As described above, the training data 205 used to train the machine learning engine 240 may include reference drug product package images including information identifying the drug product package characteristics and/or the contents contained therein. These known reference drug product package images may serve as a baseline to allow deviations from the baseline characteristics for drug product package images associated with various drug product packaging system images to be recognized by comparison with the baseline images. In accordance with various embodiments of the inventive concept, the characteristics may comprise one or more image capture light source characteristics, one or more image capture surface characteristics, one or more packaging material characteristics, and/or one or more camera characteristics. The one or more light source characteristics may comprise, for example, strength of an image capture light source, intensity of the image capture light source, and/or location of the image capture light source. The one or more image capture surface characteristics may comprise background location and/or background color. The one or more packaging material characteristics may comprise packaging material transparency, packaging material shadow, labeling color, packaging material color, and/or packaging material hot spot. The one or more camera characteristics comprise camera number, camera position, camera resolution, and/or camera image type. The AI engine 245 and/or the multi-packaging system convolutional neural network 310 may then generate a modified image of the drug product package based on the characteristics of the drug product packaging system at block 610.

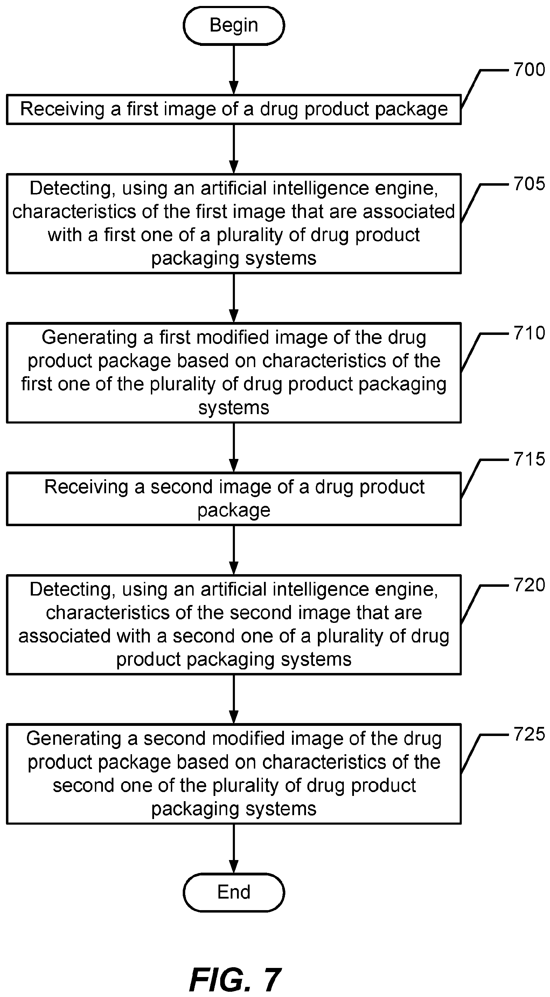

As described above, embodiments of the inventive concept may provide an AI assisted drug product package analysis system that can be used to account for variations between different drug product packaging systems through the composite packaging system module 242C, the composite packaging system module 247C and the multi-packaging system convolutional neural network 310. Thus, embodiments of the inventive concept may be used to process images of drug product packages from different drug product packaging systems and generate respective modified images that are based on the characteristics of the different drug product packaging systems, respectively. Referring now to FIG. 7, operations begin at block 700 where the AI engine 245 and/or the multi-packaging system convolutional neural network 310 receives a first image of a drug product package. At block 705, the characteristics of the first image that are associated with a first one of a plurality of drug product packaging systems may be detected using the AI engine 245 and/or the multi-packaging system convolutional neural network 310. The AI engine 245 and/or the multi-packaging system convolutional neural network 310 may then generate a first modified image of the drug product package based on the characteristics of the first one of the plurality of drug product packaging systems at block 710. The AI engine 245 and/or the multi-packaging system convolutional neural network 310 may further receive a second image of a drug product package at block 715. At block 720, the characteristics of the second image that are associated with a second one of a plurality of drug product packaging systems may be detected using the AI engine 245 and/or the multi-packaging system convolutional neural network 310. The AI engine 245 and/or the multi-packaging system convolutional neural network 310 may then generate a second modified image of the drug product package based on the characteristics of the second one of the plurality of drug product packaging systems at block 725. Similar to the embodiments of FIG. 6, the characteristics of the various ones of the plurality of drug product packaging systems may comprise one or more image capture light source characteristics, one or more image capture surface characteristics, one or more packaging material characteristics, and/or one or more camera characteristics. The one or more light source characteristics may comprise, for example, strength of an image capture light source, intensity of the image capture light source, and/or location of the image capture light source. The one or more image capture surface characteristics may comprise background location and/or background color. The one or more packaging material characteristics may comprise packaging material transparency, packaging material shadow, labeling color, packaging material color, and/or packaging material hot spot. The one or more camera characteristics comprise camera number, camera position, camera resolution, and/or camera image type.

Embodiments of the inventive concept may provide one or more AI systems that may facilitate the validation of the contents of a drug product package by generating a modified image of a drug product package based on the characteristics of the drug product packaging system, generating modified images of drug product packages with labeling content removed from one or more surfaces thereof, detecting in a drug product package image individual ones of one or more drug products contained in the drug product package, and/or identifying these drug products that have been detected in the drug product package image. The accuracy of embodiments for validating the contents of drug product packages in which labeling content has been at least partially removed including detecting individual ones of one or more drug products in the package and identifying these identified drug products by name may be further improved through taking into account the particular characteristics of the drug product packaging systems used to package the drug products when analyzing the drug product package images. Embodiments for generating modified images of drug product packages with labeling content removed from one or more surfaces thereof, detecting in a drug product package image individual ones of one or more drug products contained in the drug product package, and/or identifying these drug products that have been detected in the drug product package image are described below and in U. S. Patent Application No. 17/649,208 entitled "METHODS, SYSTEMS, AND COMPUTER PROGRAM PRODUCT FOR REMOVING EXTRANEOUS CONTENT FROM DRUG PRODUCT PACKAGING TO FACILITATE VALIDATION OF THE CONTENTS THEREIN," filed January 28, 2022.

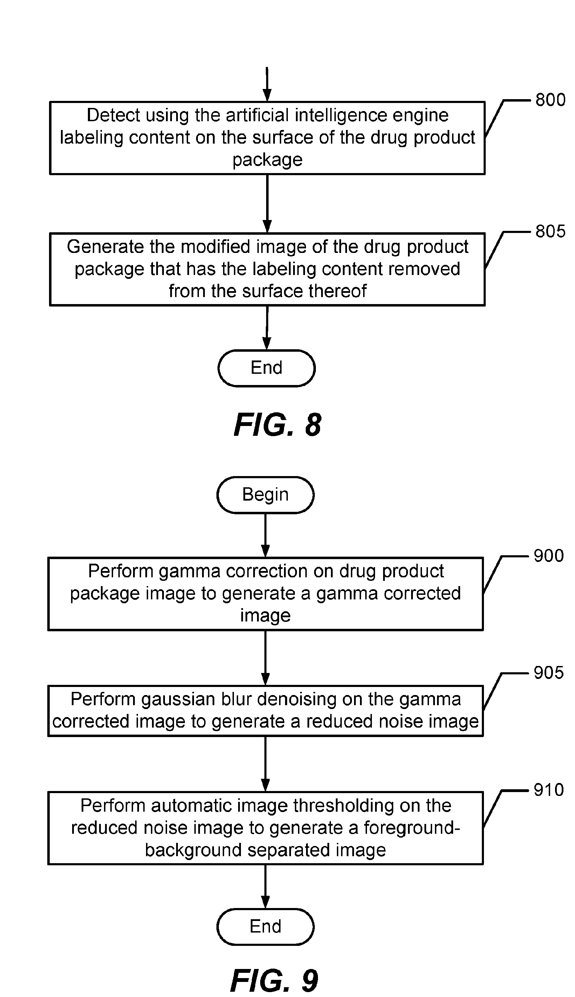

Referring now to FIG. 8, the multi-packaging system convolutional neural network 310 may receive an image of a drug product package with labeling content displayed on a surface thereof. The labeling content may include, but is not limited to, commercial marketing information, patient identification information and/or personal healthcare information (PHI). The commercial marketing information may include, for example, a logo and/or a business name. The patient identification information may include, for example, a patient name, a patient phone number, a patient address, and/or a patient identification number. The personal health care information may include, for example, names of the one or more drug products contained in the drug product package, a prescribed time of administration for each of the one or more drug products, one or more barcodes associated with the one or more drug products, a prescription order, a patient account, an identification number, and/or other information. At block 800, the labeling content on the surface of the drug product package may be detected using the multi-packaging system convolutional neural network 310. The multi-packaging system convolutional neural network 310 may then generate a modified image of the drug product package that has the labeling content removed from the surface thereof at block 805.

As described above, the drug product package image may undergo pre-processing to perform various corrections to the image data. Referring now to FIGS. 4 and 9, operations begin at block 900 where a gamma correction module 405 performs gamma correction on the drug product package image to generate a gamma corrected image. One or more cameras may make an image darker; the gamma correction may brighten the image to allow the multi-packaging system convolutional neural network 310 to better recognize the edges of various elements displayed in the image. Gamma correction may be embodied as a power law transform, except for low luminosities where it may be linear to avoid having an infinite derivative at luminance zero. This is the traditional nonlinearity applied for encoding SDR images. The exponent or "gamma," may have a value of 0.45, but the linear portion of the lower part of the curve may make the final gamma correction function to be closer to a power low exponent of 0.5, i.e., a square root transform; therefore, the gamma correction may comply with the DeVries-Rose law of brightness perception. At block 905, the gaussian blur denoising module 410 is used to perform gaussian blur denoising on the gamma corrected image to generate a reduced noise image. The gaussian blur denoising module or filter 410 may be a linear filter. It may be used to blur the image and/or to reduce the noise. Two gaussian blur denoising filters 410 may be used such that the outputs are subtracted for "unsharp masking" (edge detection). The gaussian blur denoising module or filter 410 may blur edges and reduce contrast. The Median filter is a non-linear filter that may be used as a way to reduce noise in an image. At block 910, the automatic image thresholding module 415 may perform automatic image thresholding on the reduced noise image to generate a foreground-background separated image. Thresholding is a technique used in image segmentation applications. Thresholding involves the selection of a desired gray-level threshold value for separating objects of interest in an image from the background based on their gray-level distribution. The Otsu method is a type of global thresholding that depends only on the gray value of the image. The Otsu method is a global thresholding selection method, which involves computing a gray level histogram. When applied in only one dimension an image may not be sufficiently segmented. A two-dimensional Otsu method may be used that is based on both the gray-level threshold of each pixel as well as its spatial correlation information with the neighborhood surrounding the pixel. As a result, the Otsu method may provide satisfactory segmentation when applied to noisy images. The output image from the pre-processing modules of FIG. 4 may be applied to a drug product package modification engine, such as the multi-packaging system convolutional neural network 310 of FIG. 3.

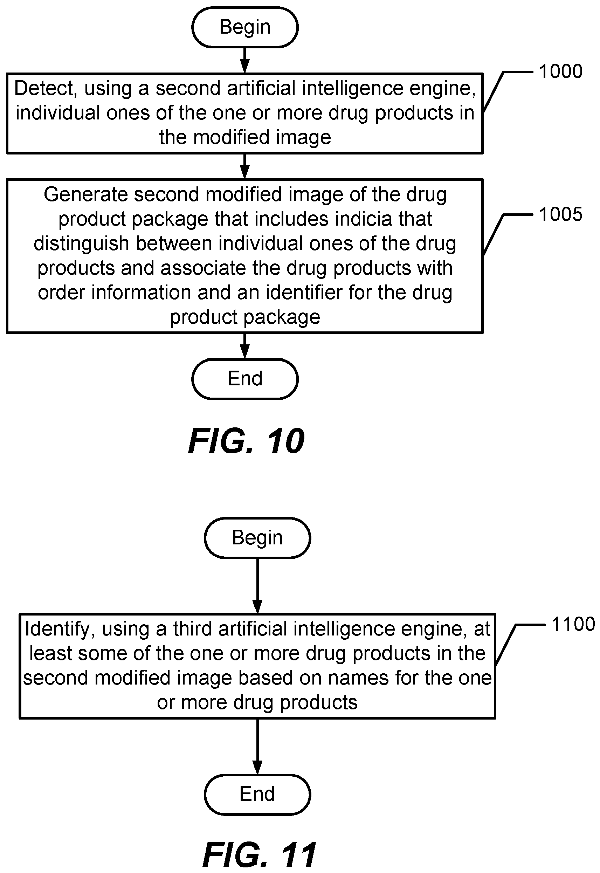

Referring now to FIG. 10, the drug product package image may be further processed to facilitate validation of the contents therein by detecting, at block 1000, individual ones of the one or more drug products in the modified image having the labeling content removed from the surface thereof. The detection may be carried out using an AI engine, such as the AI engine 245 described above with respect to FIG. 2 based on the modified drug product package image with the labeling content removed from the surface thereof along with order information for the one or more drug products contained in the drug product package and/or an identifier for the drug product package. The AI engine 245 may generate a second modified image of the drug product package that includes indicia that distinguish between individual ones of the drug products and associate the drug products with order information and an identifier for the drug product package at block 1005. In some embodiments, bounding boxes may be used as indicia that distinguish between individual ones of the drug products.

Referring now to FIG. 11, the drug product package image may be further processed to facilitate validation of the contents therein by identifying, at block 1100, at least some of the one or more drug products in the second modified image having the individual ones of the drug products detected, based on names for the one or more drug products. The identification may be carried out using an AI engine, such as the AI engine 245 described above with respect to FIG. 2 based on the modified drug product package image with the individual ones of the one or more drug products detected and order information, which includes names for the one or more drug products in the drug product package. In accordance with some embodiments of the inventive concept, the names may be associated with drug product attributes in a reference database. These attributes may include, but are not limited to, drug product shape, color, etching(s), imprint(s), weight, and/or label(s). The identified one or more drug products may further include the identification of debris, resulting from damage to a drug product that reduces all or a portion of the drug product to powder, for example. The identification of the one or more drug products in the drug product package image by name along with identifying portions of drug products and package debris may facilitate generating a count of the drug products in the drug product package for use in validating the contents of the drug product package. The drug product package image may be annotated with the names determined for the one or more drug products, but there may be circumstances where the AI engine is unable to determine a name for one or more of the drug products in the drug product package image. If there is only one or a few drug products for which a name was unable to be determined, then these drug products may be annotated with a temporary name or National Drug Code (NDC) that is new or not seen previously.

According to some embodiments of the inventive concept, the operations described above with respect to FIGS. 8 - 11 may be supplemented by basing the modified images generated by the AI system 245 and/or the multi-packaging system convolutional neural network for use in validating the contents of a drug product package on characteristics of the drug product packaging system used to package the drug product.

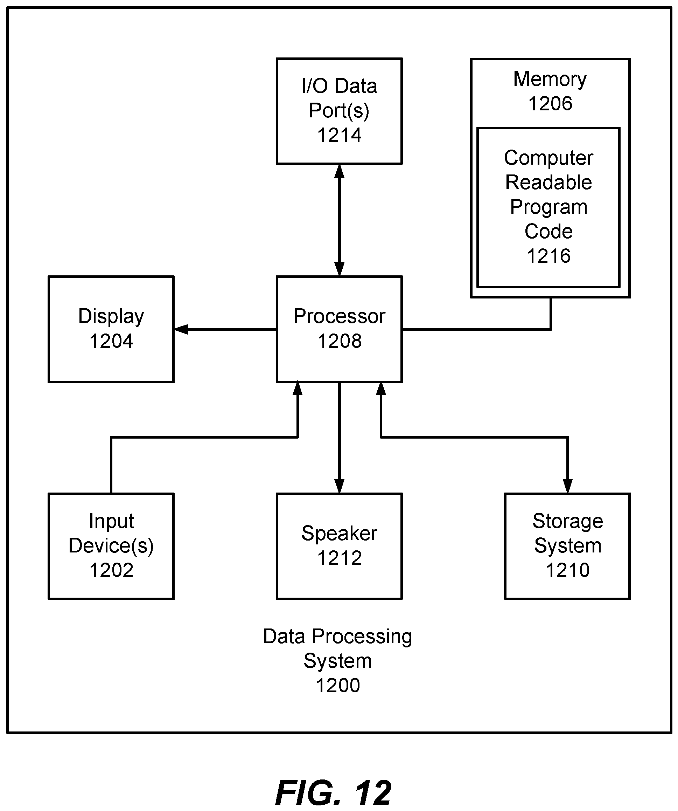

Referring now to FIG. 12, a data processing system 1200 that may be used to implement the drug product package image analysis engine(s) server 155 of FIG. 1, in accordance with some embodiments of the inventive concept, comprises input device(s) 1202, such as a keyboard or keypad, a barcode scanner, or RFID reader, a display 1204, and a memory 1206 that communicates with a processor 1208. The data processing system 1200 may further include a storage system 1210, a speaker 1212, and an input/output (I/O) data port(s) 1214 that also communicate with the processor 1208. The processor 1208 may be, for example, a commercially available or custom microprocessor. The storage system 1210 may include removable and/or fixed media, such as floppy disks, ZIP drives, hard disks, or the like, as well as virtual storage, such as a RAMDISK. The I/O data port(s) 1214 may be used to transfer information between the data processing system 1200 and another computer system or a network (e.g., the Internet). These components may be conventional components, such as those used in many conventional computing devices, and their functionality, with respect to conventional operations, is generally known to those skilled in the art. The memory 1206 may be configured with computer readable program code 1216 to facilitate AI assisted drug product package analysis to validate the drug product package contents while accounting for variations in characteristics of drug product packaging systems according to some embodiments of the inventive concept.

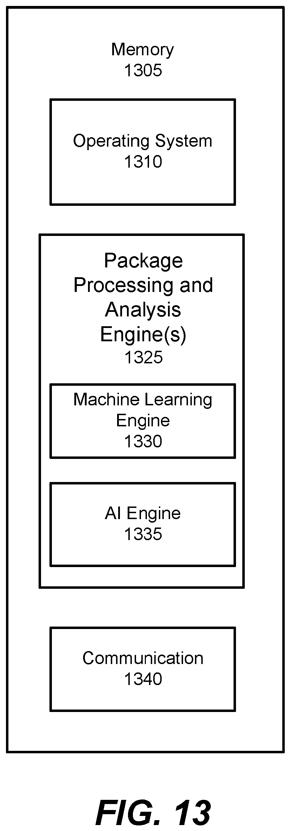

FIG. 13 illustrates a memory 1305 that may be used in embodiments of data processing systems, such as the drug product package analysis engine(s) server 155 of FIG. 1 and the data processing system 1200 of FIG. 12, respectively, to facilitate AI assisted drug product package analysis to validate the drug product package contents while accounting for variations in characteristics of drug product packaging systems according to some embodiments of the inventive concept. The memory 1305 is representative of the one or more memory devices containing the software and data used for facilitating operations of the drug product package analysis engine(s) server 155 and the drug product package analysis engine(s) module 160 as described herein. The memory 1305 may include, but is not limited to, the following types of devices: cache, ROM, PROM, EPROM, EEPROM, flash, SRAM, and DRAM. As shown in FIG. 13, the memory 1305 may contain five or more categories of software and/or data: an operating system 1310, a drug product package processing and analysis engine(s) module 1325, and a communication module 1340. In particular, the operating system 1310 may manage the data processing system's software and/or hardware resources and may coordinate execution of programs by the processor. The drug product package processing and analysis engine(s) module 1325 may comprise a machine learning engine module 1330 and an AI engine module 1335. The machine learning engine module 1330 may be configured to perform one or more operations described above with respect to the machine learning engine 240, the multi-packaging system convolutional neural network 310, and the flowcharts of FIGS. 6 - 11. The AI engine module 1335 may be configured to perform one or more operations described above with respect to the AI engine 245, the multi-packaging system convolutional neural network 310, and the flowcharts of FIGS. 6 - 11. The communication module 1340 may be configured to support communication between, for example, the drug product package analysis engine(s) server 155 and, for example, a drug product package validation system.

Although FIGS. 12 - 13 illustrate hardware/software architectures that may be used in data processing systems, such as the drug product package analysis engine(s) server 155 of FIG. 1 and the data processing system 1200 of FIG. 12, respectively, in accordance with some embodiments of the inventive concept, it will be understood that embodiments of the present invention are not limited to such a configuration but are intended to encompass any configuration capable of carrying out operations described herein.

Computer program code for carrying out operations of data processing systems discussed above with respect to FIGS. 1 - 13 may be written in a high-level programming language, such as Python, Java, C, and/or C++, for development convenience. In addition, computer program code for carrying out operations of the present invention may also be written in other programming languages, such as, but not limited to, interpreted languages. Some modules or routines may be written in assembly language or even micro-code to enhance performance and/or memory usage. It will be further appreciated that the functionality of any or all of the program modules may also be implemented using discrete hardware components, one or more application specific integrated circuits (ASICs), or a programmed digital signal processor or microcontroller.

Moreover, the functionality of the drug product package analysis engine(s) server 155 of FIG. 1 and the data processing system 1200 of FIG. 12 may each be implemented as a single processor system, a multi-processor system, a multi-core processor system, or even a network of stand-alone computer systems, in accordance with various embodiments of the inventive concept. Each of these processor/computer systems may be referred to as a "processor" or "data processing system."

The data processing apparatus described herein with respect to FIGS. 1 - 13 may be used to facilitate AI assisted drug product package analysis to validate the drug product package contents while accounting for variations characteristics of drug product packaging systems according to some embodiments of the inventive concept described herein. These apparatus may be embodied as one or more enterprise, application, personal, pervasive and/or embedded computer systems and/or apparatus that are operable to receive, transmit, process and store data using any suitable combination of software, firmware and/or hardware and that may be standalone or interconnected by any public and/or private, real and/or virtual, wired and/or wireless network including all or a portion of the global communication network known as the Internet, and may include various types of tangible, non-transitory computer readable media. In particular, the memory 1305 when coupled to a processor includes computer readable program code that, when executed by the processor, causes the processor to perform operations including one or more of the operations described herein with respect to FIGS. 1 - 13.

As described above, embodiments of the inventive concept may provide an AI assisted drug product package analysis system that may use AI technology, such as a convolutional neural network to validate the contents of a drug product package, such as a pouch or blistercard, for example, while accounting for variations in characteristics of different drug product packaging systems, which may affect the validation process due to, for example, differences in the physical packaging being used as well as the image capture system used by the different drug product packaging systems to capture images of the drug product packages. According to some embodiments of the inventive concept, the AI system may be trained to account for differences in drug product packaging system characteristics while modifying images of drug product packages to facilitate the validation of the contents contained therein. For example, AI systems according to some embodiments of the inventive concept may use a convolutional neural network to detect the labeling content on the surface of a drug product package to generate a modified image of the drug product package with the labeling content removed and one or more machine learning engines to detect and identify the drug products contained in the drug product package. this may improve the accuracy of the package validation process before, for example, a pharmacy or medical center releases packaged drug products to a customer or patient.

### Further Definitions and Embodiments:

In the above-description of various embodiments of the present disclosure, aspects of the present disclosure may be illustrated and described herein in any of a number of patentable classes or contexts including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "circuit," "module," "component," or "system." Furthermore, aspects of the present disclosure may take the form of a computer program product comprising one or more computer readable media having computer readable program code embodied thereon.

Any combination of one or more computer readable media may be used. The computer readable media may be a computer readable signal medium or a computer readable storage medium. A computer readable storage medium may be, for example, but not limited to, an electronic, magnetic, optical, electromagnetic, or semiconductor system, apparatus, or device, or any suitable combination of the foregoing. More specific examples (a non-exhaustive list) of the computer readable storage medium would include the following: a portable computer diskette, a hard disk, a random access memory (RAM), a read-only memory (ROM), an erasable programmable read-only memory (EPROM or Flash memory), an appropriate optical fiber with a repeater, a portable compact disc read-only memory (CD-ROM), an optical storage device, a magnetic storage device, or any suitable combination of the foregoing. In the context of this document, a computer readable storage medium may be any tangible medium that can contain or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device. Program code embodied on a computer readable signal medium may be transmitted using any appropriate medium, including but not limited to wireless, wireline, optical fiber cable, RF, etc., or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB.NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable instruction execution apparatus, create a mechanism for implementing the functions/acts specified in the flowchart and/or block diagram block or blocks.

These computer program instructions may also be stored in a computer readable medium that when executed can direct a computer, other programmable data processing apparatus, or other devices to function in a particular manner, such that the instructions when stored in the computer readable medium produce an article of manufacture including instructions which when executed, cause a computer to implement the function/act specified in the flowchart and/or block diagram block or blocks. The computer program instructions may also be loaded onto a computer, other programmable instruction execution apparatus, or other devices to cause a series of operational steps to be performed on the computer, other programmable apparatuses or other devices to produce a computer implemented process such that the instructions which execute on the computer or other programmable apparatus provide processes for implementing the functions/acts specified in the flowchart and/or block diagram block or blocks.

The flowchart and block diagrams in the figures illustrate the architecture, functionality, and operation of possible implementations of systems, methods, and computer program products according to various aspects of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The terminology used herein is for the purpose of describing particular aspects only and is not intended to be limiting of the disclosure. As used herein, the singular forms "a", "an" and "the" are intended to include the plural forms as well, unless the context clearly indicates otherwise. It will be further understood that the terms "comprises," "comprising," "include", "including", "includes", "have", "has", "having", or variants thereof when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Like reference numbers signify like elements throughout the description of the figures.

It will also be understood that, although the terms first, second, etc. may be used herein to describe various elements, these elements should not be limited by these terms. These terms are only used to distinguish one element from another.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

The description of the present disclosure has been presented for purposes of illustration and description, but is not intended to be exhaustive or limited to the disclosure in the form disclosed. Many modifications and variations will be apparent to those of ordinary skill in the art without departing from the scope of the disclosure. The aspects of the disclosure herein were chosen and described in order to best explain the principles of the disclosure and the practical application, and to enable others of ordinary skill in the art to understand the disclosure with various modifications as are suited to the particular use contemplated.

## Claims

1. A method for validating drug product package content, comprising:
receiving (600) an image of a drug product package that contains one or more drug products therein;
detecting (605), using an artificial intelligence engine (245) that is trained on a plurality of drug product packaging system specific characteristics sets that are associated with a plurality of drug product packaging systems, respectively, characteristics of the image that are specific to a first drug product packaging system of the plurality of drug product packaging systems; and
generating (610) a modified image of the drug product package based on the characteristics of the first drug product packaging system.

2. The method of Claim 1, wherein the characteristics of the first drug product packaging system comprise one or more image capture light source characteristics, one or more image capture surface characteristics, one or more packaging material characteristics, and/or one or more camera characteristics;
wherein the one or more light source characteristics comprise strength of an image capture light source, intensity of the image capture light source, and/or location of the image capture light source;
wherein the one or more image capture surface characteristics comprise background location and/or background color;
wherein the one or more packaging material characteristics comprise packaging material transparency, packaging material shadow, labeling color, packaging material color, and/or packaging material hot spot; and
wherein the one or more camera characteristics comprise camera number, camera position, camera resolution, and/or camera image type.

3. The method of Claim 1, wherein the image is a first image and the modified image is a first modified image, the method further comprising:
receiving (715) a second image of a second drug product package that contains one or more drug products therein;
detecting (720), using the artificial intelligence engine, characteristics of the second image that are specific to a second drug product packaging system of the plurality of drug product packaging systems; and
generating (725) a second modified image of the second drug product package based on the characteristics of the second drug product packaging system.

4. The method of Claim 1, further comprising:
detecting (800), using the artificial intelligence engine, labeling content on a surface of the drug product package;
wherein generating the modified image of the drug product package comprises generating (805) a modified image of the drug product package that has the labeling content removed from the surface thereof.

5. The method of Claim 4, wherein the artificial intelligence engine is a first artificial intelligence engine and the modified image is a first modified image, the method further comprising:
receiving order information for the one or more drug products and an identifier for the drug product package;
detecting (1000), using a second artificial intelligence engine, individual ones of the one or more drug products in the first modified image; and
generating (1005) a second modified image of the drug product package that includes indicia that distinguish between the individual ones of the one or more drug products and associate the one or more drug products with the order information and the identifier for the drug product package.

6. The method of Claim 5, wherein the order information comprises names for the one or more drug products in the drug product package, the method further comprising:
identifying (1100), using a third artificial intelligence engine, at least some of the one or more drug products in the second modified image based on the names for the one or more drug products;
wherein the names are associated with drug product attributes in a reference database.

7. The method of Claim 4, further comprising:
performing gamma correction (900) on the image of the drug product package responsive to receiving the image of the drug product package to generate a gamma corrected image of the drug product package;
performing gaussian blur denoising (905) on the gamma corrected image of the drug product package to generate a reduced noise image of the drug product package; and
performing automatic image thresholding (910) on the reduced noise image of the drug product package to generate a foreground-background separated image of the drug product package;
wherein detecting the labeling content comprises:
detecting, using the artificial intelligence engine, the labeling content on a surface of the foreground-background separated image of the drug product package.

8. A system for validating drug product package content, comprising:
a processor; and
a memory coupled to the processor and comprising computer readable program code embodied in the memory that is executable by the processor to perform operations comprising:
receiving (600) an image of a drug product package that contains one or more drug products therein;
detecting (605), using an artificial intelligence engine that is trained on a plurality of drug product packaging system specific characteristics sets that are associated with a plurality of drug product packaging systems, respectively, characteristics of the image that are specific to a first drug product packaging system of the plurality of drug product packaging systems; and
generating (610) a modified image of the drug product package based on the characteristics of the first drug product packaging system.

9. The system of Claim 8, wherein the characteristics of the first drug product packaging system comprise one or more image capture light source characteristics, one or more image capture surface characteristics, one or more packaging material characteristics, and/or one or more camera characteristics;
wherein the one or more light source characteristics comprise strength of an image capture light source, intensity of the image capture light source, and/or location of the image capture light source;
wherein the one or more image capture surface characteristics comprise background location and/or background color;
wherein the one or more packaging material characteristics comprise packaging material transparency, packaging material shadow, labeling color, packaging material color, and/or packaging material hot spot; and
wherein the one or more camera characteristics comprise camera number, camera position, camera resolution, and/or camera image type.

10. The system of Claim 8, wherein the image is a first image and the modified image is a first modified image, the operations further comprising:
receiving (715) a second image of a second drug product package that contains one or more drug products therein;
detecting (720), using the artificial intelligence engine, characteristics of the second image that are specific to a second drug product packaging system of the plurality of drug product packaging systems; and
generating (725) a second modified image of the second drug product package based on the characteristics of the second drug product packaging system.

11. A computer program product for validating drug product package content, comprising:
a non-transitory computer readable storage medium comprising computer readable program code embodied in the medium that is executable by a processor to perform operations comprising:
receiving (600) an image of a drug product package that contains one or more drug products therein;
detecting (605), using an artificial intelligence engine that is trained on a plurality of drug product packaging system specific characteristics sets that are associated with a plurality of drug product packaging systems, respectively, characteristics of the image that are specific to a first drug product packaging system of the plurality of drug product packaging systems; and
generating (610) a modified image of the drug product package based on the characteristics of the first drug product packaging system.

## Patentansprüche

1. Verfahren zum Validieren von Inhalt einer Arzneimittelproduktpackung, umfassend:
Empfangen (600) eines Bildes einer Arzneimittelproduktpackung, die ein oder mehrere Arzneimittelprodukte darin enthält;
Erkennen (605), unter Verwendung einer Engine künstlicher Intelligenz (245), die mit einer Vielzahl von für Arzneimittelproduktverpackungssysteme spezifischen Merkmalssätzen trainiert ist, die jeweils einer Vielzahl von Arzneimittelproduktverpackungssystemen zugeordnet sind, von Merkmalen des Bildes, die für ein erstes Arzneimittelproduktverpackungssystem der Vielzahl von Arzneimittelproduktverpackungssystemen spezifisch sind; und
Erzeugen (610) eines modifizierten Bildes der Arzneimittelproduktpackung basierend auf den Merkmalen des ersten Arzneimittelproduktverpackungssystems.

2. Verfahren nach Anspruch 1, wobei die Merkmale des ersten Arzneimittelproduktverpackungssystems ein oder mehrere Bilderfassungslichtquellenmerkmale, ein oder mehrere Bilderfassungsoberflächenmerkmale, ein oder mehrere Verpackungsmaterialmerkmale und/oder ein oder mehrere Kameramerkmale umfassen;
wobei das eine oder die mehreren Lichtquellenmerkmale eine Stärke einer Bilderfassungslichtquelle, eine Intensität der Bilderfassungslichtquelle und/oder eine Lage der Bilderfassungslichtquelle umfassen;
wobei das eine oder die mehreren Bilderfassungsoberflächenmerkmale eine Hintergrundlage und/oder eine Hintergrundfarbe umfassen;
wobei das eine oder die mehreren Verpackungsmaterialmerkmale eine Verpackungsmaterialtransparenz, einen Verpackungsmaterialschatten, eine Etikettierungsfarbe, eine Verpackungsmaterialfarbe und/oder einen Verpackungsmaterial-Hotspot umfassen; und
wobei das eine oder die mehreren Kameramerkmale eine Kameranummer, eine Kameraposition, eine Kameraauflösung und/oder eine Kamerabildart umfassen.

3. Verfahren nach Anspruch 1, wobei das Bild ein erstes Bild ist und das modifizierte Bild ein erstes modifiziertes Bild ist, das Verfahren ferner umfassend:
Empfangen (715) eines zweiten Bildes einer zweiten Arzneimittelproduktpackung, die ein oder mehrere Arzneimittelprodukte darin enthält;
Erkennen (720), unter Verwendung der Engine künstlicher Intelligenz, von Merkmalen des zweiten Bildes, die für ein zweites Arzneimittelproduktverpackungssystem der Vielzahl von Arzneimittelproduktverpackungssystemen spezifisch sind; und
Erzeugen (725) eines zweiten modifizierten Bildes der zweiten Arzneimittelproduktpackung basierend auf den Merkmalen des zweiten Arzneimittelproduktverpackungssystems.

4. Verfahren nach Anspruch 1, ferner umfassend:
Erkennen (800), unter Verwendung der Engine künstlicher Intelligenz, von Etikettierungsinhalt auf einer Oberfläche der Arzneimittelproduktpackung;
wobei das Erzeugen des modifizierten Bildes der Arzneimittelproduktpackung das Erzeugen (805) eines modifizierten Bildes der Arzneimittelproduktpackung, in dem der Etikettierungsinhalt von der Oberfläche davon entfernt ist, umfasst.

5. Verfahren nach Anspruch 4, wobei die Engine künstlicher Intelligenz eine erste Engine künstlicher Intelligenz ist und das modifizierte Bild ein erstes modifiziertes Bild ist, das Verfahren ferner umfassend:
Empfangen von Bestellinformationen für das eine oder die mehreren Arzneimittelprodukte und eines Identifikators für die Arzneimittelproduktpackung;
Erkennen (1000), unter Verwendung einer zweiten Engine künstlicher Intelligenz, einzelner des einen oder der mehreren Arzneimittelprodukte in dem ersten modifizierten Bild; und
Erzeugen (1005) eines zweiten modifizierten Bildes der Arzneimittelproduktpackung, das Kennzeichnungen einschließt, die zwischen den einzelnen des einen oder der mehreren Arzneimittelprodukte unterscheiden und das eine oder die mehreren Arzneimittelprodukte den Bestellinformationen und dem Identifikator für die Arzneimittelproduktpackung zuordnen.

6. Verfahren nach Anspruch 5, wobei die Bestellinformationen Namen für das eine oder die mehreren Arzneimittelprodukte in der Arzneimittelproduktpackung umfassen, das Verfahren ferner umfassend:
Identifizieren (1100), unter Verwendung einer dritten Engine künstlicher Intelligenz, mindestens einiger des einen oder der mehreren Arzneimittelprodukte in dem zweiten modifizierten Bild basierend auf den Namen für das eine oder die mehreren Arzneimittelprodukte;
wobei die Namen Arzneimittelproduktattributen in einer Referenzdatenbank zugeordnet sind.

7. Verfahren nach Anspruch 4, ferner umfassend:
Durchführen einer Gammakorrektur (900) an dem Bild der Arzneimittelproduktpackung als Reaktion auf das Empfangen des Bildes der Arzneimittelproduktpackung, um ein gammakorrigiertes Bild der Arzneimittelproduktpackung zu erzeugen;
Durchführen einer Gaußschen Weichzeichnungsentrauschung (905) an dem gammakorrigierten Bild der Arzneimittelproduktpackung, um ein rauschreduziertes Bild der Arzneimittelproduktpackung zu erzeugen; und
Durchführen einer automatischen Bildschwellenwertbildung (910) an dem rauschreduzierten Bild der Arzneimittelproduktpackung, um ein in Vordergrund und Hintergrund getrenntes Bild der Arzneimittelproduktpackung zu erzeugen;
wobei das Erkennen des Etikettierungsinhalts umfasst:
Erkennen, unter Verwendung der Engine künstlicher Intelligenz, des Etikettierungsinhalts auf einer Oberfläche des in Vordergrund und Hintergrund getrennten Bildes der Arzneimittelproduktpackung.

8. System zum Validieren von Inhalt einer Arzneimittelproduktpackung, umfassend:
einen Prozessor; und
einen an den Prozessor gekoppelten Speicher, umfassend computerlesbaren Programmcode, der in dem Speicher implementiert und durch den Prozessor ausführbar ist, um Abläufe durchzuführen, umfassend:
Empfangen (600) eines Bildes einer Arzneimittelproduktpackung, die ein oder mehrere Arzneimittelprodukte darin enthält;
Erkennen (605), unter Verwendung einer Engine künstlicher Intelligenz, die mit einer Vielzahl von für Arzneimittelproduktverpackungssysteme spezifischen Merkmalssätzen trainiert ist, die jeweils einer Vielzahl von Arzneimittelproduktverpackungssystemen zugeordnet sind, von Merkmalen des Bildes, die für ein erstes Arzneimittelproduktverpackungssystem der Vielzahl von Arzneimittelproduktverpackungssystemen spezifisch sind; und
Erzeugen (610) eines modifizierten Bildes der Arzneimittelproduktpackung basierend auf den Merkmalen des ersten Arzneimittelproduktverpackungssystems.

9. System nach Anspruch 8, wobei die Merkmale des ersten Arzneimittelproduktverpackungssystems ein oder mehrere Bilderfassungslichtquellenmerkmale, ein oder mehrere Bilderfassungsoberflächenmerkmale, ein oder mehrere Verpackungsmaterialmerkmale und/oder ein oder mehrere Kameramerkmale umfassen;
wobei das eine oder die mehreren Lichtquellenmerkmale die Stärke einer Bilderfassungslichtquelle, die Intensität der Bilderfassungslichtquelle und/oder die Lage der Bilderfassungslichtquelle umfassen;
wobei das eine oder die mehreren Bilderfassungsoberflächenmerkmale die Hintergrundlage und/oder die Hintergrundfarbe umfassen;
wobei das eine oder die mehreren Verpackungsmaterialmerkmale die Verpackungsmaterialtransparenz, den Verpackungsmaterialschatten, die Etikettierungsfarbe, die Verpackungsmaterialfarbe und/oder den Verpackungsmaterial-Hotspot umfassen; und
wobei das eine oder die mehreren Kameramerkmale die Kameranummer, die Kameraposition, die Kameraauflösung und/oder die Kamerabildart umfassen.

10. System nach Anspruch 8, wobei das Bild ein erstes Bild ist und das modifizierte Bild ein erstes modifiziertes Bild ist, die Abläufe ferner umfassend:
Empfangen (715) eines zweiten Bildes einer zweiten Arzneimittelproduktpackung, die ein oder mehrere Arzneimittelprodukte darin enthält;
Erkennen (720), unter Verwendung der Engine künstlicher Intelligenz, von Merkmalen des zweiten Bildes, die für ein zweites Arzneimittelproduktverpackungssystem der Vielzahl von Arzneimittelproduktverpackungssystemen spezifisch sind; und
Erzeugen (725) eines zweiten modifizierten Bildes der zweiten Arzneimittelproduktpackung basierend auf den Merkmalen des zweiten Arzneimittelproduktverpackungssystems.

11. Computerprogrammprodukt zum Validieren von Inhalt einer Arzneimittelproduktpackung, umfassend:
ein nichtflüchtiges computerlesbares Speicherungsmedium, umfassend in dem Medium implementierten computerlesbaren Programmcode, der durch einen Prozessor ausführbar ist, um Abläufe durchzuführen, umfassend:
Empfangen (600) eines Bildes einer Arzneimittelproduktpackung, die ein oder mehrere Arzneimittelprodukte darin enthält;
Erkennen (605), unter Verwendung einer Engine künstlicher Intelligenz, die mit einer Vielzahl von für Arzneimittelproduktverpackungssysteme spezifischen Merkmalssätzen trainiert ist, die jeweils einer Vielzahl von Arzneimittelproduktverpackungssystemen zugeordnet sind, von Merkmalen des Bildes, die für ein erstes Arzneimittelproduktverpackungssystem der Vielzahl von Arzneimittelproduktverpackungssystemen spezifisch sind; und
Erzeugen (610) eines modifizierten Bildes der Arzneimittelproduktpackung basierend auf den Merkmalen des ersten Arzneimittelproduktverpackungssystems.

## Revendications

1. Procédé de validation du contenu d'un emballage de produit médicamenteux, comprenant :
la réception (600) d'une image d'un emballage de produit médicamenteux qui contient un ou plusieurs produits médicamenteux en son sein ;
la détection (605), à l'aide d'un moteur d'intelligence artificielle (245) qui est entraîné sur une pluralité d'ensembles de caractéristiques spécifiques à un système de conditionnement de produit médicamenteux qui sont associés à une pluralité de systèmes de conditionnement de produit médicamenteux, respectivement, de caractéristiques de l'image qui sont spécifiques à un premier système de conditionnement de produit médicamenteux de la pluralité de systèmes de conditionnement de produit médicamenteux ; et
la génération (610) d'une image modifiée de l'emballage de produit médicamenteux sur la base des caractéristiques du premier système de conditionnement de produit médicamenteux.

2. Procédé selon la revendication 1, dans lequel les caractéristiques du premier système de conditionnement de produit médicamenteux comprennent une ou plusieurs caractéristiques de source de lumière de capture d'image, une ou plusieurs caractéristiques de surface de capture d'image, une ou plusieurs caractéristiques de matériau d'emballage et/ou une ou plusieurs caractéristiques de caméra ;
dans lequel les une ou plusieurs caractéristiques de source de lumière comprennent la puissance d'une source de lumière de capture d'image, l'intensité de la source de lumière de capture d'image et/ou l'emplacement de la source de lumière de capture d'image ;
dans lequel les une ou plusieurs caractéristiques de surface de capture d'image comprennent l'emplacement d'arrière-plan et/ou la couleur d'arrière-plan ;
dans lequel les une ou plusieurs caractéristiques de matériau d'emballage comprennent la transparence du matériau d'emballage, l'ombre du matériau d'emballage, la couleur d'étiquetage, la couleur du matériau d'emballage et/ou un point chaud du matériau d'emballage ; et
dans lequel les une ou plusieurs caractéristiques de caméra comprennent le nombre de caméras, la position de caméra, la résolution de caméra et/ou le type d'image de caméra.

3. Procédé selon la revendication 1, dans lequel l'image est une première image et l'image modifiée est une première image modifiée, le procédé comprenant en outre :
la réception (715) d'une seconde image d'un second emballage de produit médicamenteux qui contient un ou plusieurs produits médicamenteux en son sein ;
la détection (720), à l'aide du moteur d'intelligence artificielle, de caractéristiques de la seconde image qui sont spécifiques à un second système de conditionnement de produit médicamenteux de la pluralité de systèmes de conditionnement de produit médicamenteux ; et
la génération (725) d'une seconde image modifiée du second emballage de produit médicamenteux sur la base des caractéristiques du second système de conditionnement de produit médicamenteux.

4. Procédé selon la revendication 1, comprenant en outre :
la détection (800), à l'aide du moteur d'intelligence artificielle, d'un contenu d'étiquetage sur une surface de l'emballage de produit médicamenteux ;
dans lequel la génération de l'image modifiée de l'emballage de produit médicamenteux comprend la génération (805) d'une image modifiée de l'emballage de produit médicamenteux dont le contenu d'étiquetage a été retiré de la surface de celui-ci.

5. Procédé selon la revendication 4, dans lequel le moteur d'intelligence artificielle est un premier moteur d'intelligence artificielle et l'image modifiée est une première image modifiée, le procédé comprenant en outre :
la réception d'informations de commande pour les un ou plusieurs produits médicamenteux et d'un identifiant pour l'emballage de produit médicamenteux ;
la détection (1000), à l'aide d'un deuxième moteur d'intelligence artificielle, de produits individuels parmi les un ou plusieurs produits médicamenteux dans la première image modifiée ; et
la génération (1005) d'une seconde image modifiée de l'emballage de produit médicamenteux qui comprend des indices qui distinguent les produits individuels parmi les un ou plusieurs produits médicamenteux et associent les un ou plusieurs produits médicamenteux aux informations de commande et à l'identifiant pour l'emballage de produit médicamenteux.

6. Procédé selon la revendication 5, dans lequel les informations de commande comprennent des noms pour les un ou plusieurs produits médicamenteux dans l'emballage de produit médicamenteux, le procédé comprenant en outre :
l'identification (1100), à l'aide d'un troisième moteur d'intelligence artificielle, d'au moins certains des un ou plusieurs produits médicamenteux dans la seconde image modifiée sur la base des noms pour les un ou plusieurs produits médicamenteux ;
dans lequel les noms sont associés à des attributs de produit médicamenteux dans une base de données de référence.

7. Procédé selon la revendication 4, comprenant en outre :
l'exécution d'une correction gamma (900) sur l'image de l'emballage de produit médicamenteux en réponse à la réception de l'image de l'emballage de produit médicamenteux pour générer une image corrigée en gamma de l'emballage de produit médicamenteux ;
l'exécution d'un débruitage par flou gaussien (905) sur l'image corrigée en gamma de l'emballage de produit médicamenteux pour générer une image à bruit réduit de l'emballage de produit médicamenteux ; et
l'exécution d'un seuillage automatique d'image (910) sur l'image à bruit réduit de l'emballage de produit médicamenteux pour générer une image à premier plan et arrière-plan séparés de l'emballage de produit médicamenteux ;
dans lequel la détection du contenu d'étiquetage comprend :
la détection, à l'aide du moteur d'intelligence artificielle, du contenu d'étiquetage sur une surface de l'image à premier plan et arrière-plan séparés de l'emballage de produit médicamenteux.

8. Système de validation du contenu d'un emballage de produit médicamenteux, comprenant :
un processeur ; et
une mémoire couplée au processeur et comprenant un code de programme lisible par ordinateur incorporé dans la mémoire qui est exécutable par le processeur pour réaliser des opérations comprenant :
la réception (600) d'une image d'un emballage de produit médicamenteux qui contient un ou plusieurs produits médicamenteux en son sein ;
la détection (605), à l'aide d'un moteur d'intelligence artificielle qui est entraîné sur une pluralité d'ensembles de caractéristiques spécifiques à un système de conditionnement de produit médicamenteux qui sont associés à une pluralité de systèmes de conditionnement de produit médicamenteux, respectivement, de caractéristiques de l'image qui sont spécifiques à un premier système de conditionnement de produit médicamenteux de la pluralité de systèmes de conditionnement de produit médicamenteux ; et
la génération (610) d'une image modifiée de l'emballage de produit médicamenteux sur la base des caractéristiques du premier système de conditionnement de produit médicamenteux.

9. Système selon la revendication 8, dans lequel les caractéristiques du premier système de conditionnement de produit médicamenteux comprennent une ou plusieurs caractéristiques de source de lumière de capture d'image, une ou plusieurs caractéristiques de surface de capture d'image, une ou plusieurs caractéristiques de matériau d'emballage et/ou une ou plusieurs caractéristiques de caméra ;
dans lequel les une ou plusieurs caractéristiques de source de lumière comprennent la puissance d'une source de lumière de capture d'image, l'intensité de la source de lumière de capture d'image et/ou l'emplacement de la source de lumière de capture d'image ;
dans lequel les une ou plusieurs caractéristiques de surface de capture d'image comprennent l'emplacement d'arrière-plan et/ou la couleur d'arrière-plan ;
dans lequel les une ou plusieurs caractéristiques de matériau d'emballage comprennent la transparence du matériau d'emballage, l'ombre du matériau d'emballage, la couleur d'étiquetage, la couleur du matériau d'emballage et/ou un point chaud du matériau d'emballage ; et
dans lequel les une ou plusieurs caractéristiques de caméra comprennent le nombre de caméras, la position de caméra, la résolution de caméra et/ou le type d'image de caméra.

10. Système selon la revendication 8, dans lequel l'image est une première image et l'image modifiée est une première image modifiée, les opérations comprenant en outre :
la réception (715) d'une seconde image d'un second emballage de produit médicamenteux qui contient un ou plusieurs produits médicamenteux en son sein ;
la détection (720), à l'aide du moteur d'intelligence artificielle, de caractéristiques de la seconde image qui sont spécifiques à un second système de conditionnement de produit médicamenteux de la pluralité de systèmes de conditionnement de produit médicamenteux ; et
la génération (725) d'une seconde image modifiée du second emballage de produit médicamenteux sur la base des caractéristiques du second système de conditionnement de produit médicamenteux.

11. Produit-programme informatique pour valider le contenu d'un emballage de produit médicamenteux, comprenant :
un support de stockage non transitoire lisible par ordinateur comprenant un code de programme lisible par ordinateur incorporé dans le support qui est exécutable par un processeur pour réaliser des opérations comprenant :
la réception (600) d'une image d'un emballage de produit médicamenteux qui contient un ou plusieurs produits médicamenteux en son sein ;
la détection (605), à l'aide d'un moteur d'intelligence artificielle qui est entraîné sur une pluralité d'ensembles de caractéristiques spécifiques à un système de conditionnement de produit médicamenteux qui sont associés à une pluralité de systèmes de conditionnement de produit médicamenteux, respectivement, de caractéristiques de l'image qui sont spécifiques à un premier système de conditionnement de produit médicamenteux de la pluralité de systèmes de conditionnement de produit médicamenteux ; et
la génération (610) d'une image modifiée de l'emballage de produit médicamenteux sur la base des caractéristiques du premier système de conditionnement de produit médicamenteux.
